(19) Europäisches Patentamt
European Patent Office
Office européen des brevets

(11) Veröffentlichungsnummer: **0 068 373**
**A1**

(12) **EUROPÄISCHE PATENTANMELDUNG**

(21) Anmeldenummer: **82105401.2**

(22) Anmeldetag: **19.06.82**

(51) Int. Cl.³: **C 07 C 119/06**, C 07 C 101/04

(30) Priorität: **01.07.81 DE 3125872**

(43) Veröffentlichungstag der Anmeldung: **05.01.83**
**Patentblatt 83/1**

(84) Benannte Vertragsstaaten: **AT BE CH DE FR GB IT LI NL**

(71) Anmelder: **BASF Aktiengesellschaft,**
**Carl-Bosch-Strasse 38, D-6700 Ludwigshafen (DE)**

(72) Erfinder: **Schulz, Guenter, Dr., An der Froschlache 3, D-6700 Ludwigshafen (DE)**
Erfinder: **Buschmann, Ernst, Dr., Georg-Ludwig-Krebs-Strasse 10, D-6700 Ludwigshafen (DE)**
Erfinder: **Zeeh, Bernd, Dr., Thorwaldsenstrasse 5, D-6700 Ludwigshafen (DE)**

(54) **Schiff-Basen von Aminocycloalkancarbonsäureestern, Verfahren zu ihrer Herstellung sowie ihre Verwendung als Zwischenprodukte.**

(57) Die vorliegende Erfindung betrifft neue Schiff-Basen von Aminocycloalkancarbonsäureestern der Formel

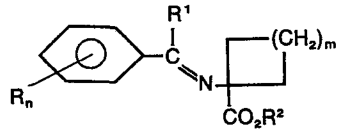

I,

worin R, $R^1$, $R^2$, m und n die in der Beschreibung angegebene Bedeutung besitzen, sowie deren Herstellung. Die Verbindungen sind Zwischenprodukte zur Herstellung der entsprechenden Aminocycloalkancarbonsäuren bzw. von deren Estern.

EP 0 068 373 A1

# Schiff-Basen von Aminocycloalkancarbonsäureestern, Verfahren zu ihrer Herstellung sowie ihre Verwendung als Zwischenprodukte

Die vorliegende Erfindung betrifft neue Schiff-Basen von Aminocycloalkancarbonsäureestern, ein Verfahren zu ihrer Herstellung und deren Verwendung als Zwischenprodukte zur Herstellung von weitgehend bekannten Aminocycloalkancarbonsäureestern und Aminocycloalkancarbonsäuren (vgl. D.H. Rich und J.P. Tam. Synth. 1978, 46; U. Schöllkopf, D. Hoppe und R. Jentsch, Chem. Ber. 108, 1580 (1975); I. Bregovec und T. Jakovcić, Monatshefte 103, 288 (1972)), die wachstumsregulierende Wirkstoffe sind, oder zu solchen umgesetzt werden können (vgl. DE-OS 23 42 229).

Es ist bereits bekannt, daß man wachstumsregulatorisch wirksame Aminocycloalkancarbonsäuren erhält, wenn man Isocyanessigsäureester mit alpha,omega-Dihalogenalkanen cycloalkyliert und die so erhaltenen Isocyancycloalkancarbonsäureester verseift (DE-OS 28 24 517). Dieser Syntheseweg weist jedoch einige Nachteile auf. Besonders hervorzuheben sind dabei der unangenehme Geruch und die Toxizität der flüchtigen Verbindungen vom Isonitriltyp (M.P. Periasamy und H.M. Walborsky, Organic Preparations and Procedures Int. 11 (6), 293 - 311 (1979).

Es ist außerdem bekannt, daß die einfach herstell- und handhabbaren Schiff-Basen von Glycinestern unter Verwendung von sehr starken Basen wie Lithiumdiisopropylamid mit Alkyliodiden dialkyliert werden können (G. Stork, M.Y.W. Leong und A.M. Touzin, J. Org. Chem. 41, 3491). Die Alkylierung von Anionen von N-Arylidenglycinestern mit nicht aktivierten Alkylbromiden oder Alkylchloriden galt

WK/P

jedoch bislang als nicht möglich (B. Bey und J.P. Vevert, Tetrahedron Lett. 1977, 1455 - 1458).

Es wurden nun einfache Zwischenprodukte gefunden, die sich gut zur Herstellung von Aminocycloalkancarbonsäuren und deren Estern eignen.

Gegenstand der Erfindung sind Schiff-Basen von Aminocycloalkancarbonsäureestern der Formel I

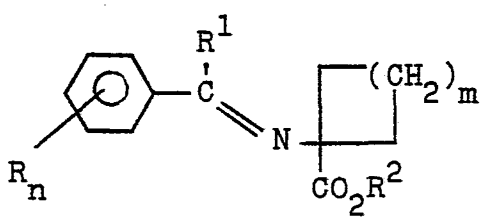

I,

in welcher

R für Wasserstoff, Halogen, Alkyl, Alkoxy, Alkylthiol, Alkylsulfonyl, Halogenalkyl, Nitro, Cyano, gegebenenfalls substituiertes Phenyl oder gegebenenfalls substituiertes Phenoxy,

$R^1$ für Wasserstoff, Alkyl, Alkenyl, Alkinyl, gegebenenfalls substituiertes Cycloalkylalkyl oder gegebenenfalls substituiertes Aryl,

$R^2$ für Alkyl, Alkenyl, Alkinyl, gegebenenfalls substituiertes Cycloalkylalkyl, gegebenenfalls substituiertes Arylalkyl oder gegebenenfalls substituiertes Aryl,

n für die ganzen Zahlen 1, 2 oder 3,

m für die ganzen Zahlen von 0 bis 8 stehen,

und im Cycloalkylring 1 oder 2 Wasserstoffatome durch Alkyl, Alkenyl, Alkinyl, gegebenenfalls substituiertes Cycloalkylalkyl, gegebenenfalls substituiertes Arylalkyl oder gegebenenfalls substituiertes Aryl ersetzt sein können.

In der Formel I steht R vorzugsweise für Wasserstoff, für die Halogene Fluor, Chlor und Brom, für Nitro und Cyano, ferner vorzugsweise für Alkyl und Alkylsulfonyl mit jeweils 1 bis 4 Kohlenstoffatomen, für Alkoxy und Alkylthio mit 1 bis 2 Kohlenstoffatomen sowie für Halogenalkyl mit bis zu 4 Kohlenstoffatomen und bis zu 5 Halogenatomen, insbesondere mit bis zu 2 Kohlenstoffatomen und bis zu 3 gleichen oder verschiedenen Halogenatomen, wobei als Halogenatome vorzugsweise Fluor und Chlor in Betracht kommen und als Beispiel für Halogenalkyl speziell Trifluormethyl genannt sei. R steht außerdem vorzugsweise für gegebenenfalls ein- oder mehrfach, gleichartig oder verschieden substituiertes Phenyl oder Phenoxy, wobei als Substituenten vorzugsweise in Frage kommen: Halogen, insbesondere Fluor, Chlor und Brom; Cyano, Nitro sowie Halogenalkyl mit bis zu 2 Kohlenstoffatomen und bis zu 3 gleichen oder verschiedenen Halogenatomen, wobei als Halogenatome vorzugsweise Fluor und Chlor in Betracht kommen und als Beispiel für Halogenalkyl speziell Trifluormethyl genannt sei.

$R^1$, $R^2$ und sowie die Substituenten am Cycloalkylring stehen jeweils unabhängig voneinander vorzugsweise für Wasserstoff (mit Ausnahme von $R^2$), Alkyl, Alkenyl und Alkinyl mit jeweils bis zu 4 Kohlenstoffatomen; für Cycloalkylalkyl mit 5 bis 7 Kohlenstoffatomen im Cycloalkylteil, wobei der Cycloalkylteil gegebenenfalls durch Alkyl mit 1 bis 4 Kohlenstoffatomen substituiert sein kann sowie gegebenenfalls ein- oder mehrfach, gleichartig oder verschieden substituiertes Benzyl (mit Ausnahme von $R^1$) oder verschieden substituiertes Phenyl, wobei als Substituenten vorzugsweise in Frage kommen: Halogen, insbesondere Fluor, Chlor und Brom; Cyano, Nitro, Acetylamino, Alkyl mit bis zu 4 Kohlenstoffatomen, Phenyl, Phenoxy und Halogenalkyl mit bis zu 2 Kohlenstoffatomen und bis zu 3 gleichen oder verschiedenen Halogenatomen, wobei als Halogenatome insbesondere

Fluor und Chlor in Betracht kommen und als Beispiel für Halogenalkyl speziell Trifluormethyl genannt sei. n steht vorzugsweise für die Zahlen 1 oder 2, m vorzugsweise für die Zahlen von 0 bis 6.

Im einzelnen seien die Verbindungen der folgenden Tabelle genannt.

| $R_n$ | $R^1$ | 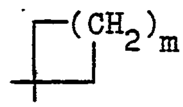 | $R^2$ |
|---|---|---|---|
| H | H | 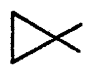 | $C_2H_5$ |
| 2-Cl | H | 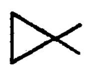 | $C_2H_5$ |
| 4-Cl | H | 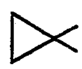 | $C_2H_5$ |
| 2,4-$Cl_2$ | H | 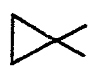 | $C_2H_5$ |
| 4-$NO_2$ | H | 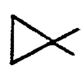 | $C_2H_5$ |
| 2,4-$(NO_2)_2$ | H | 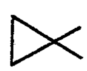 | $C_2H_5$ |
| 4-Phenoxy | H | 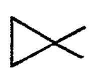 | $C_2H_5$ |
| 3-$CF_3$ | H | 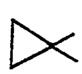 | $C_2H_5$ |

| $R_n$ | $R^1$ | 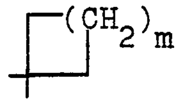 | $R^2$ |
|---|---|---|---|
| H | $CH_3$ | 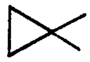 | $C_2H_5$ |
| $2,4-Cl_2$ | $CH_3$ | 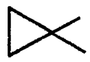 | $C_2H_5$ |
| $3,4-Cl_2$ | $CH_3$ | 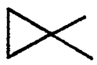 | $C_2H_5$ |
| $2,5-Cl_2$ | $CH_3$ | 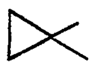 | $C_2H_5$ |
| H | $C_6H_5$ | 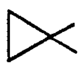 | $C_2H_5$ |
| H | $C_6H_5$ | 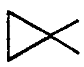 | $C_2H_5$ |
| 4-Cl | $4-Cl-C_6H_5$ | 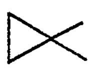 | $C_2H_5$ |
| H | H | 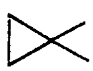 | tert.$C_4H_9$ |
| H | H | 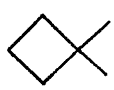 | $C_2H_5$ |
| H | H | 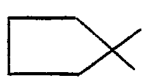 | $C_2H_5$ |

| $R_n$ | $R^1$ | 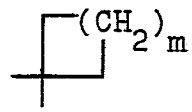 | $R^2$ |
|---|---|---|---|
| H | H | 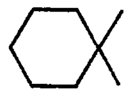 | $C_2H_5$ |
| H | H | 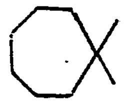 | $C_2H_5$ |
| H | H | 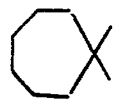 | $C_2H_5$ |
| H | H | 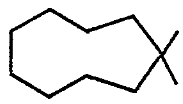 | $C_2H_5$ |
| H | H | 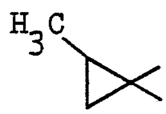 | $C_2H_5$ |
| H | H | 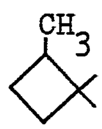 | $C_2H_5$ |
| H | H | 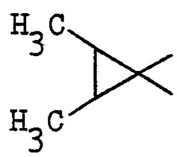 | $C_2H_5$ |
| H | H | 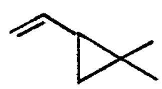 | $C_2H_5$ |

Überraschenderweise erhält man die Schiff-Basen der allgemeinen Formel I, indem man Aminoessigsäurederivate der Formel II

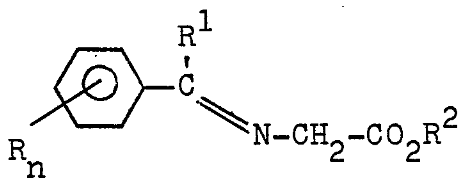

II,

worin R, $R^1$, $R^2$ und n die oben angegebene Bedeutung haben, mit alpha,omega-disubstituierten Alkanen der Formel III

$$X-CH_2-(CH_2)_m-CH_2-Y \qquad III,$$

worin m die oben angegebene Bedeutung hat, die Alkylengruppe wie oben angegeben substituiert sein kann und X und Y jeweils unabhängig voneinander Cl, Br, J. O-Tosyl oder O-Mesyl bedeuten, in Gegenwart einer Base umsetzt.

Verwendet man beispielsweise N-Benzylidenglycin-ethylester und 1,2-Dibromethan als Ausgangsstoffe, so kann der Reaktionsablauf durch das folgende Formelschema wiedergegeben werden:

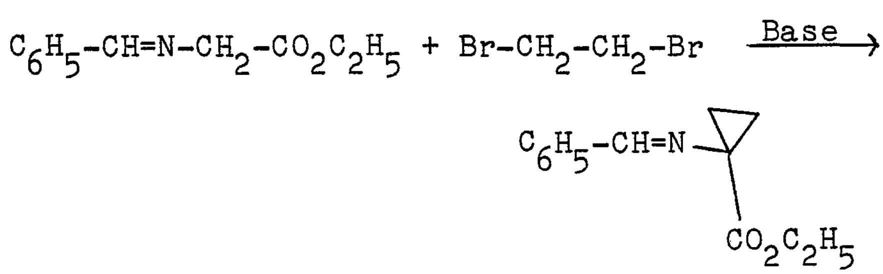


Die erfindungsgemäße Umsetzung wird vorzugsweise in Gegenwart eines Verdünnungsmittels durchgeführt. Als solche kommen insbesondere Gemische von Dimethylsulfoxid und Ether in Frage, vorzugsweise im Mischungsverhältnis von 1 : 2 bis 1 : 3. Als Basen eignen sich Verbindungen der Formel MeR,

worin Me ein Alkalimetall, vorzugsweise Natrium oder Kalium, und R ein Wasserstoffatom oder einen $O-C_{2-5}$-Alkylrest bedeuten. Bevorzugt sind Kaliumtertiärbutylat und Natriumhydrid geeignet. Die Reaktionstemperaturen können in einem größeren Bereich variiert werden. Im allgemeinen arbeitet man zwischen 0 und 50°C, vorzugsweise zwischen 20 und 35°C.

Bei der Durchführung des erfindungsgemäßen Verfahrens setzt man auf 1 Mol Glycinester-Schiff-Base II vorzugsweise 1 Mol des alpha,omega-disubstituierten Alkans III und vorzugsweise 2 bis 2,2 Mol Base ein. Die Isolierung der Verbindungen der Formel I erfolgt nach üblichen Methoden.

Die neuen Verbindungen der Formel I sind interessante Zwischenprodukte zur Herstellung von Aminocycloalkancarbonsäureestern der Formel IV

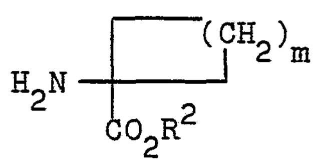

IV

und Aminocycloalkancarbonsäuren der Formel V

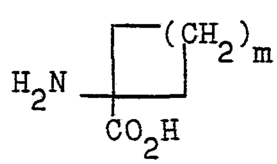

V,

worin $R^2$, $R^3$ und m die oben angegebene Bedeutung haben und der Cycloalkylring wie angegeben substituiert sein kann.

Die zum Teil bekannten Verbindungen IV und V sind Wirkstoffe mit wachstumsregulatorischer Wirkung. Sie lassen sich aus den Schiff-Basen I leicht und in vorteilhafter Weise herstellen.

Die Aminocycloalkancarbonsäureester IV erhält man, indem man die Schiff-Basen I mit überschüssiger wäßriger Säure gegebenenfalls in Gegenwart eines Lösungsvermittlers hydrolysiert. Nachdem die bei der Hydrolyse mitangefallenen Aldehyde oder Ketone von den wäßrigen Phasen abgetrennt worden sind, werden durch Zusatz eines Säurebinders die Aminosäureester IV freigesetzt. Ihre Isolierung erfolgt nach üblichen Methoden.

Als Säuren können wäßrige organische Säuren wie zum Beispiel Ameisensäure, Essigsäure, Oxalsäure oder Zitronensäure oder verdünnte wäßrige Mineralsäuren wie zum Beispiel Salzsäure, Schwefelsäure, Salpetersäure oder Phosphorsäure verwendet werden. Vorzugsweise wird das 2- bis 10fache der theoretisch benötigten Säuremenge eingesetzt.

Als Lösungsmittler kommen gegebenenfalls inerte organische Lösungsmittel, die mit Wasser mischbar sind, wie zum Beispiel Nitrile, wie Acetonitril, Alkohole, wie Methanol, Ethanol oder Propanol, oder Ether, wie Tetrahydrofuran oder Dioxan, in Frage.

Als Säurebinder kann man alle üblicherweise verwendbaren anorganischen oder organischen Säurebinder zugeben, vorzugsweise finden Alkalicarbonate, wie zum Beispiel Natriumcarbonat, Natriumhydrogencarbonat oder Kaliumcarbonat Verwendung.

Die Reaktionstemperaturen können in einem größeren Bereich variiert werden. Im allgemeinen arbeitet man zwischen 0 und 100°C, vorzugsweise bei 20 bis 30°C.

Die Verbindungen der Formel V erhält man, indem man die Schiff-Basen I oder die Ester IV mit wäßrigen Basen gegebenenfalls in Gegenwart eines Lösungsvermittlers hydrolysiert. Die Isolierung der Aminocycloalkancarbonsäuren erfolgt, nachdem gegebenenfalls bei der Hydrolyse mitangefallene Aldehyde oder Ketone von der wäßrigen Phase abgetrennt worden sind und nachdem neutralisiert worden ist, nach üblichen Methoden.

Als wäßrige Basen kommen wäßrige Lösungen von Alkali- oder Erdalkalihydroxiden wie zum Beispiel Kaliumhydroxid, Natriumhydroxid, Calciumhydroxid oder Bariumhydroxid in Frage, vorzugsweise sei Bariumhydroxid genannt.

Zur Neutralisation kommen vorzugsweise wäßrige Mineralsäuren wie Salzsäure, Schwefelsäure, Salpetersäure oder Phosphorsäure in Frage, besonders bevorzugt sei Schwefelsäure genannt. Die Kombination der Hydrolyse mit Bariumhydroxid und Neutralisation mit Schwefelsäure, ermöglicht nach Abtrennen von Bariumsulfat in einfacher Weise die salzfreie Isolation der Verbindungen V.

Als Lösungsvermittler kommen gegebenenfalls inerte organische Lösungsmittel, die mit Wasser mischbar sind, in Frage, wie zum Beispiel Nitrile wie Acetonitril, Alkohole wie Methanol, Ethanol oder Propanol, oder Ether wie Tetrahydrofuran oder Dioxan.

Die Reaktionstemperaturen können in einem größeren Bereich varriert werden. Im allgemeinen arbeitet man zwischen 0 und 100°C, vorzugsweise bei 50 bis 100°C.

Die als Ausgangsmaterial verwendeten Schiff-Basen der Glycinester der Formel II sind zum Teil bekannt (vgl. z.B.: M.J. O'Donnell, J.M. Boniece und S.E. Earp, Tetrahedron Lett. 1978, 2641; P. Bey und J.P. Vevert, Tetrahedron Lett. 1977, 1455; O. Gerngross und A. Olcay, Chem. Ber. 96, 2550 (1963); G. Stork, A.Y.W. Leong und A.M. Touzin,

J. Org. Chem 41, 3491 (1976) oder können nach den gängigen Verfahren aus Aminosäureestern und entsprechenden Ketonen bzw. Aldehyden durch Kondensation unter Wasseraustritt gewonnen werden (vgl. Methoden der Organischen Chemie (Houben-Weyl), E. Müller, Ed., Bd. XV, 1, Kap. 31, Seite 230 oder "The Chemistry of the Carbon Nitrogen Double Bond", S. Patai, Ed. Interscience, New York 1970).

Als Beispiele für die Schiff-Basen II seien genannt:

| $R_n$ | $R^1$ | $R^2$ |
|---|---|---|
| H | H | $C_2H_5$ |
| 2-Cl | H | $C_2H_5$ |
| 4-Cl | H | $C_2H_5$ |
| 2,4-$Cl_2$ | H | $C_2H_5$ |
| 4-$NO_2$ | H | $C_2H_5$ |
| 2,4-$(NO_2)_2$ | H | $C_2H_5$ |
| 4-Phenoxy | H | $C_2H_5$ |
| 3-$CF_3$ | H | $C_2H_5$ |
| H | $CH_3$ | $C_2H_5$ |
| 2,4-$Cl_2$ | $CH_3$ | $C_2H_5$ |
| 3,4-$Cl_2$ | $CH_3$ | $C_2H_5$ |
| H | $C_6H_5$ | $C_2H_5$ |
| 4-Cl | 4-Cl-$C_6H_4$ | $C_2H_5$ |
| H | H | tert.-$C_4H_9$ |

Die weiterhin als Ausgangsstoffe zu verwendenden alpha,omega-disubstituierten Alkane der Formel III sind allgemein bekannte Verbindungen der organischen Chemie. Bevorzugte Verbindungen der Formel III sind die, in denen X und Y unabhängig voneinander Cl, Br oder o-Tosyl bedeuten. Als Beispiele seien genannt: 1,2-Dibromethan, 1,2-Dichlorethan, 1,2-Dichlorpropan, 1,3-Dibrompropan, 1,4-Dibrombutan, 3,4-Dichlorbuten, 1,3-Dichlorbutan, 2,3-Dichlorbutan, 1,5-Dibrompentan, 1,6-Dibromhexan, 1,7-Dibromheptan, 1,8-Dibromoctan.

In den Herstellungsbeispielen wird die Herstellung der neuen Zwischenprodukte der Formel I und deren Verwendung gezeigt.

Herstellungsbeispiele

Beispiel 1

N-Benzyliden-1-amino-cyclopropancarbonsäure-ethylester

100 g (0,523 mol) N-Benzylidenglycin-ethylester und 45,1 ml (0,523 mol) 1,2-Dibromethan in 500 trockenem Diethylether und 170 ml trockenem Dimethylsulfoxid werden unter Stickstoff portionsweise so mit 25,2 g (1,05 mol) Natriumhydrid versetzt, daß die Temperatur des gut durchmischten Ansatzes zwischen 20 und 30°C bleibt. Nach Rühren über Nacht bei Raumtemperatur wird filtriert und das Filtrat mit 1 l Wasser versetzt. Man trennt die etherische Phase ab, wäscht diese zweimal mit je 200 ml Wasser, trocknet über Natriumsulfat und erhält nach Eindampfen im Vakuum 80 g (70 %) N-Benzyliden-1-aminocyclopropansäure-ethylester als Öl.

$^1H$-NMR ($CDCl_3$): $\delta$ = 1,2 - 1,9, m [4]; 1,28 t, J = 7 Hz. [3]; 4,23, q, J = 7 Hz [2]; 7,25 - 7,95, m [5]; 8,40, s [1] verbreitert.

Die Verbindung kann durch Verseifen in die Aminocyclopropancarbonsäure übergeführt werden, Fp.: 229 - 231°C.

Beispiel 2

N-Benzyliden-1-aminocyclopropancarbonsäure-ethylester

200 g (1,05 mol) N-Benzylidenglycin-ethylester und 90,5 ml (1,05 mol) 1,2-Dibromethan in 900 ml trockenem Diethylether und 300 l trockenem Dimethylsulfoxid werden portionsweise so mit 260 g (2,3 mol) Kaliumtertiärbutylat versetzt, daß die Temperatur des gut durchmischten Ansatzes zwischen 25 und 35°C bleibt. Nach Rühren über Nacht bei Raumtemperatur wird durch Zusetzen von 4 N Salzsäure überschüssige Base neutralisiert. Man erweitert mit 1,5 l Wasser und trennt die etherische Phase ab, die dann zweimal mit je 300 ml Wasser gewaschen wird und nach Trocknen mit Natriumsulfat und Eindampfen 155 g (68 %) N-Benzyliden-1-aminocyclopropancarbonsäure-ethylester als Öl liefert.

Das $^1H$-NMR-Spektrum entspricht dem der Substanz des Beispiels 1.

Beispiel 3

N-Benzyliden-1-aminocyclopentancarbonsäure-ethylester

Ausgehend von 100 g (0,523 mol) N-Benzylidenglycin-ethylester und 62,1 ml (0,523 mol) 1,4-Dibrombutan erhält man analog Beispiel 2 in 450 ml Ether und 150 ml Dimethylsulfoxid mit 130 g (1,15 mol) Kaliumtertiärbutylat als

Base 117 g (91 %) N-Benzylidencyclopentancarbonsäure-ethylester als Öl. Charakteristische $^1$H-NMR-Signale ($CDCl_3$): $\delta$= 1,6 - 2,5, m [8]; 8,20, s [1] verbreitert.

Die Verbindung kann durch Verseifen in die Aminocyclopentancarbonsäure übergeführt werden, Fp.: 320 - 322°C (Zersetzung).

Beispiel 4

N-Benzyliden-1-aminocycloheptancarbonsäureethylester

Ausgehend von 200 g (1,05 mol) N-Benzylidenglycin-ethylester und 170ml (1,05 mol) 1,6-Dibromhexan erhält man analog Beispiel 2 258 g (90 %) N-Benzyliden-1-aminocycloheptancarbonsäureethylester als Öl.

Charakteristische $^1$H-NMR-Signale ($CDCl_3$): $\delta$= 1,0 - 2,3, m [12+3]; 8,27, s [1] verbreitert. Die Verbindung kann durch Verseifen in die Aminocycloheptancarbonsäure übergeführt werden, Fp.: 350°C (Zersetzung).

Beispiel 5

N-Benzyliden-1-aminocyclooctancarbonsäure-ethylester

Ausgehend von 100 g (0,523 mol) N-Benzylidenglycin-ethylester und 89,9 ml (0,523 mol) 1,7-Dibromheptan erhält man analog Beispiel 1, 138 g (92 %) N-Benzyliden-1-aminocyclooctancarbonsäure-ethylester als Öl.

Charakteristische $^1$H-NMR-Signale ($CDCl_3$): $\delta$= 0,9 - 2,2, m [14+3]; 8,27, s [1] verbreitert. Die Verbindung kann durch Verseifen in die Aminocyclooctancarbonsäure übergeführt werden, Fp.: 310 - 316°C (Zersetzung).

Verwendungsbeispiele

Beispiel A

a) 1-Aminocyclopropancarbonsäureethylester

100 g (0,46 mol) N-Benzyliden-1-aminocyclopropancarbonsäure-ethylester (Beispiel 1) werden mit 500 ml 4 N Salzsäure kräftig verrührt. Nach Abtrennen der Benzaldehyd-Phase wird mit Natriumcarbonat alkalisch gemacht und mit Methylenchlorid extrahiert. Die mit Natriumsulfat getrocknete organische Phase liefert beim Eindampfen 50 g (84 %) 1-Amino-cyclopropancarbonsäure-ethylester als Öl. Sdp.: $34^{o}C$/0,6 mbar. $^{1}H$-NMR ($CDCl_3$): $\delta$ = 0,8 - 1,4, m [4]; 1,26, t, J = 7 Hz. [3]; s [2] verbreitert; 4,13, q, J = 7 Hz. [2].

b) 1-Aminocyclopropancarbonsäure

30 g (0,232 mol) 1-Aminocyclopropancarbonsäure-ethylester werden in 300 ml Wasser und 200 ml Ethanol mit 36,6 g (0,116 mol) Bariumhydroxid-Octahydrat 2 h bei Rückflußtemperatur gerührt. Nach Abdampfen des Ethanols wird durch Zusatz von 0,116 mol Schwefelsäure Bariumsulfat gefällt. Nach Dekantieren und Eindampfen der wäßrigen Lösung erhält man 22,3 g (95 %) 1-Aminocyclopropancarbonsäure, Fp.: 229 - $231^{o}C$.

Beispiel B

a) 1-Aminocyclopentancarbonsäureethylester

Ausgehend von 100 g (0,41 mol) N-Benzyliden-1-aminocyclopentancarbonsäure-ethylester (Beispiel 3) erhält man analog Beispiel A beschrieben, 54,5 g (85 %)

1-Aminocyclopentancarbonsäureethylester, Fp. (Hydrochlorid): 165 - 190°C (Zersetzung).

b) 1-Aminocyclopentancarbonsäure

36,4 g (0,232 mol) des 1-Aminocyclopentancarbonsäureethylesters liefern analog Beispiel A, b) 28,5 g (95 %) 1-Aminocyclopentancarbonsäure, Fp.: 320 - 322°C (Zersetzung).

Beispiel C

a) 1-Aminocycloheptancarbonsäureethylester

Ausgehend von 100 g (0,37 mol) N-Benzyliden-1-aminocycloheptancarbonsäureethylester (Beispiel 4) erhält man analog Beispiel 6 61 g (90 %) 1-Aminocycloheptancarbonsäureethylester als Öl.

b) 1-Aminocycloheptancarbonsäure

43 g (0,232 mol) 1-Aminocycloheptancarbonsäureethylester liefern analog Beispiel A, b) 33,9 g (93 %) 1-Aminocycloheptancarbonsäure, Fp.: 310 - 312°C (Zersetzung).

Beispiel D

a) 1-Aminocyclooctancarbonsäure-ethylester

Ausgehend von 100 g (0,35 mol) N-Benzyliden-1-aminocyclooctancarbonsäure-ethylester erhält man analog Beispiel 6 60,6 g (87 %) 1-Aminocyclooctancarbonsäure-ethylester als Öl. Fp. (Hydrochlorid): 165°C (Zersetzung).

b) 46,2 g (0,232 mol) 1-Aminocyclooctancarbonsäureethylester liefern analog Beispiel A, b) 38,1 g (96 %) 1-Aminocyclooctancarbonsäure, Fp.: 310 - 316°C (Zersetzung).

## Patentansprüche

1. Schiff-Basen von Aminocycloalkancarbonsäureestern der Formel I

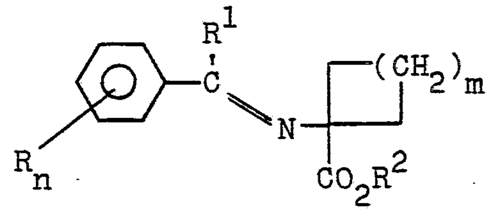

I,

in welcher
R für Wasserstoff, Halogen, Alkyl, Alkoxy, Alkylthiol, Alkylsulfonyl, Halogenalkyl, Nitro, Cyano, gegebenenfalls substituiertes Phenyl oder gegebenenfalls substituiertes Phenoxy,
$R^1$ für Wasserstoff, Alkyl, Alkenyl, Alkinyl, gegebenenfalls substituiertes Cycloalkylalkyl oder gegebenenfalls substituiertes Aryl,
$R^2$ für Alkyl, Alkenyl, Alkinyl, gegebenenfalls substituiertes Cycloalkylalkyl, gegebenenfalls substituiertes Arylalkyl oder gegebenenfalls substituiertes Aryl,
n für die ganzen Zahlen 1, 2 oder 3,
m für die ganzen Zahlen von 0 bis 8 stehen,
und im Cycloalkylring 1 oder 2 Wasserstoffatome durch Alkyl, Alkenyl, Alkinyl, gegebenenfalls substituiertes Cycloalkylalkyl, gegebenenfalls substituiertes Arylalkyl oder gegebenenfalls substituiertes Aryl ersetzt sein können.

2. N-Benzylidencyclopropancarbonsäure-ethylester.

3. Verfahren zur Herstellung der Verbindungen der Formel I gemäß Anspruch 1, <u>dadurch gekennzeichnet</u>, daß man Aminoessigsäurederivate der Formel II

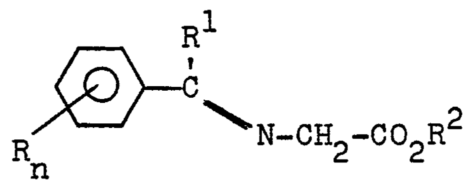

II,

worin R, $R^1$, $R^2$ und n die oben angegebene Bedeutung haben, mit alpha,omega-disubstituierten Alkanen der Formel III

$$X-CH_2-(CH_2)_m-CH_2-Y \qquad III,$$

worin m die oben angegebene Bedeutung hat, die Alkylengruppe wie oben angegeben substituiert sein kann und X und Y jeweils unabhängig voneinander Cl, Br, J, O-Tosyl oder O-Mesyl bedeuten, in Gegenwart einer Base umsetzt.

4. Verwendung der Verbindungen der Formel I gemäß Anspruch 1 zur Herstellung der entsprechenden Aminocycloalkancarbonsäure bzw. deren Estern.

Europäisches Patentamt

# EUROPÄISCHER RECHERCHENBERICHT

0068373

Nummer der Anmeldung

EP 82 10 5401

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE-A-2 624 290 (CHINOIN GYOGYSZER) * Beispiel 15 * ----- | 1-4 | C 07 C 119/06<br>C 07 C 101/04 |

RECHERCHIERTE SACHGEBIETE (Int. Cl. ³)

C 07 C 119/00

Der vorliegende Recherchenbericht wurde für alle Patentansprüche erstellt.

| Recherchenort | Abschlußdatum der Recherche | Prüfer |
|---|---|---|
| DEN HAAG | 08-10-1982 | GAUTIER R.H.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82